# EUROPEAN PATENT SPECIFICATION

(11) **EP 2 592 085 B1**
(45) Date of publication and mention of the grant of the patent: **27.07.2016**
(21) Application number: 11188961.4
(22) Date of filing: 14.11.2011
(51) Int. Cl.: C07D 513/04, A61K 31/551, A61P 25/08

(54) **Thiazolo[3,2-a][1,3]diazepine derivatives and pharmaceutical compositions containing the same as anticonvulsant agents and method for their preparation**
Thiazolo[3,2-a][1,3]diazepin-Derivate und diese enthaltende pharmazeutische Zusammensetzungen als Antikonvulsiva und Verfahren zu ihrer Herstellung
Dérivés de thiazolo[3,2-a][1,3]diazépine et compositions pharmaceutiques contenant ces dérivés en tant qu'agents anticonvulsants et procédé pour leur préparation

(43) Date of publication of application: 15.05.2013
(73) Proprietor: King Saud University, 11421 Riyadh (SA)
(72) Inventor: Al-Rashood, Sara T. A., Riyadh 11333 (SA); El-Subbagh, Hussein I., 11421 Riyadh (SA); Hassan, Ghada S., Riyadh 11333 (SA); El-Taher, Kamal E. H., Riyadh 11333 (SA); Al-Omar, Mohamed A., 11421 Riyadh (SA)
(74) Representative: Goddar, Heinz J.

(56) References cited:
- DE-A1- 10 320 732
- H. I. EL-SUBBAGH ET AL.: "Synthesis and anticonvulsant activity of some new thiazolo[3,2-a][1,3]diazepine, benzo[d]thiazolo[5,2-a][12,6]diazepine and benzo[d]oxazolo[5,2-a][12,6]diazepine analogues", EUROPEAN JOURNAL OF MEDICINAL CHEMISTRY, vol. 46, no. 11, November 2011 (2011-11), pages 5567-5572, XP002670614, ISSN: 0223-5234, DOI: 10.1016/j.ejmech.2011.09.21
- EL-SUBBAGH ET AL: "New ultra-short acting hypnotic: Synthesis, biological evaluation, and metabolic profile of ethyl 8-oxo-5,6,7,8-tetrahydro-thiazolo[3,2-a][1 ,3]diazepin-3-carboxylate (HIE-124)", BIOORGANIC & MEDICINAL CHEMISTRY LETTERS, PERGAMON, ELSEVIER SCIENCE, GB, vol. 18, no. 1, 7 November 2007 (2007-11-07), pages 72-77, XP022410859, ISSN: 0960-894X, DOI: 10.1016/J.BMCL.2007.11.011
- KADI ADNAN A ET AL: "Synthesis, ultra-short acting hypnotic activity, and metabolic profile of ethyl 8-oxo-5,6,7,8-tetrahydro-thiazolo[3,2- a][1,3]diazepin-3-carboxylate (HIE-124)", ARCHIV DER PHARMAZIE, WILEY - VCH VERLAG GMBH & CO. KGAA, DE, vol. 341, no. 2, 1 January 2008 (2008-01-01), pages 81-89, XP009148692, ISSN: 0365-6233, DOI: 10.1002/ARDP.200700132
- QUARTARONE SILVANA ET AL: "Synthesis and anticonvulsant activity of N-3 substituted 2,3-benzodiazepines", FARMACO, SOCIETA CHIMICA ITALIANA, PAVIA, IT, vol. 59, 1 January 2004 (2004-01-01), pages 353-358, XP002489054, ISSN: 0014-827X, DOI: 10.1016/J.FARMAC.2004.01.005
- A. KADI ET AL.: "Liquid chromatographic high-throughput analysis of the new ultra-short acting hypnotic 'HIE-124' and its metabolite in mice serum using a monolithic silica column", ANALYST, vol. 136, no. 3, 7 February 2011 (2011-02-07), pages 591-597, XP002670615,
- ROGAWSKI ET AL: "Therapeutic potential of excitatory amino acid antagonists: channel blockers and 2,3-benzodiazepines", TRENDS IN PHARMACOLOGICAL SCIENCES, ELSEVIER, HAYWARTH, GB, vol. 14, no. 9, 1 September 1993 (1993-09-01), pages 325-331, XP023843277, ISSN: 0165-6147, DOI: 10.1016/0165-6147(93)90005-5 [retrieved on 1993-09-01]

## Description

The present invention relates to compounds useful as anticonvulsant agents, pharmaceutical composition containing the compounds as well as a method for their preparation.

Epilepsy is one of the most common neurological disorders, which is characterized by seizures that take various forms and result from episodic neural discharges. The forms of seizures depend on the part of the brain affected. Epilepsy affects 0.5-1% of the population. Although there is no recognizable cause which can be identified, patient may develop seizure after brain trauma, infection or tumor growth, or other kinds of neurological diseases, including various inherited neurological syndromes. Epilepsy is treated mainly with drugs, though brain surgery may be used for severe cases. Current antiepileptic drugs are effective in controlling seizures in about 70% of patients, but their use is often limited by side-effects (Bordie M.J., Lancet. 1990, 336, 350-354; Shoravon S.D., Lancet. 1990, 336, 93-96; Meldrum B.S. et al. In New Anticonvulsant Drugs. Current Problems in Epilepsy 4; John Libby: London, 1986. Flaherty P.T. et al., J. Med. Chem.1996, 39, 1509-1513).

GABA (γ-aminobutyric acid) is the principal inhibitory transmitter of many CNS pathways regulating numerous neurological functions including convulsions, anxiety and sleep activity. GABA acts on the GABA_{A} chloride ion channel. Molecular biology studies have demonstrated that several different receptor subunits combine to form the GABA_{A} receptor complex with a number of receptor subtypes such as benzodiazepine receptor (BzR). Ligands acting at the BzR allosteric binding site of the GABA_{A} receptor modulate the action of GABA on chloride ion flux. They show a wide variety of pharmacological actions ranging from full agonistic effects such as sedative/hypnotic, anxiolytic and anticonvulsant activities; to inverse agonistic effects such as anxiogenic, and proconvulsant activities. Several classes of chemical compounds such as benzodiazepines, steroids and barbiturates bind to the GABA_{A} chlorine ion channel complex. The azepine derivatives such as imidazo[2,1-*b*]thiazepine exhibited affinity for BzR. Their profile of action was characterized as partially agonistic at the BzRs on the basis of the GABA shift.

The α-Amino-3-hydroxy-5-methyl-4-isoxazole propionate receptors (AMPARs) are glutamate-gated ion channels which mediate the majority of fast excitatory synaptic transmissions in the mammalian brain. Hyperactivation of AMPARs is implicated in a broad range of acute and chronic neurodegenerative and neuropsychiatric conditions such as epilepsy. Thus, the modulation of AMPARs as potential targets for therapeutic intervention in many neurological disorders is one of the goals in neuropharmacology (Dingledine R. et al. Pharmacol. Rev. 1999, 51, 7-61; Lees G. J., Drugs 2000, 59, 33-78). Consequently, several antagonists of AMPAR such as talampanel (Rogawski M. A. Trends Pharmacol. Sci. 1993, 14, 325-331) and CFM-2 (Quartarone S., et al. IL Farmaco 2004, 59, 353-358) have been reported and showed promising therapeutic potential for the prevention and treatment of epilepsy.

H. I. EI-Subbagh et al., European Journal of Medicinal Chemistry, vol. 46, # 11, pg. 5567-5572, Nov. 2011 discloses thiazolo[3,2-a][1,3]diazepines useful for treating convulsions.

DE 103 20 732 relates to 3-carboxylic acid diazepine derivatives and its use as rapid-acting anasthetics. The publication of Kadi et al. analyst, 2011, 136, 591-597 relates to liquid chromatographic high-throughput analysis of a new ultra short acting hypnotic 3-carboxylic acid diazepine derivative and its metabolic in mice serum using a monolithic silica column.

It is an object of the present invention to provide an anticonvulsant agent which overcomes the drawbacks of the prior art. Especially a compound shall be provided exhibiting potent in *vivo* anticonvulsant activity. Additionally, a pharmaceutical composition containing such anticonvulsant agent shall be provided, as well as a method for its preparation

This object is achieved by a compound according to formula 1: 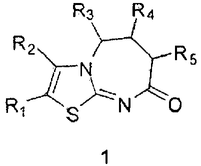 wherein R₁, R₂, R₃, R₄ and R₅ are each independently selected from the group consisting of hydrogen, halogen, C₁-C₂₀-alkyl, C₁-C20-haloalkyl, C₁-C₂₀-alkoxy, C₁-C₂₀-haloalkoxy, aryl, heteroaryl, mercapto, alkylthio, amino, alkylamino, or wherein R₂, R₃ and R₄ are a member of a ring system.

In an preferred embodiment, R₂ is selected from the group consisting of hydrogen, mercapto and C₁-C₂₀-alkyl, preferably methyl, ethyl, propyl isopropyl or butyl, halogen or amino.

Even preferred, R₃ is hydrogen or is taken together with R₄ to form an, optionally substituted, alicyclic, aryl, or heteroaryl ring system.

Preferably, R₁, R₂, R₃, R₄ and R₅ are independently selected from aryl, more preferably phenyl and naphthyl, or heteroaryl, more preferably furyl, pyrrolyl, thienyl, imidazolyl, thiadiazolyl, pyridinyl, pyridazinyl, pyrimidinyl, benzothiazolyl, and oxadiazolyl.

Preferred, the compound is present in form of its addition salt, preferably hydrochloride, hydrobromide, phosphate, nitrate, acetate, malate, succinate, fumarate, tartrate, salicylate, sorbate, lactate, p-toluene sulphate, or naphthalene-1,5-disulfonate salts.

The object is further achieved by a method for preparing an inventive compound, comprising: reacting a compound according to formula 2 with a compound according to formula 3 to prepare a compound according to formula 4, and reacting the compound of formula 4 to result in a compound of formula 1, as given in scheme 1: 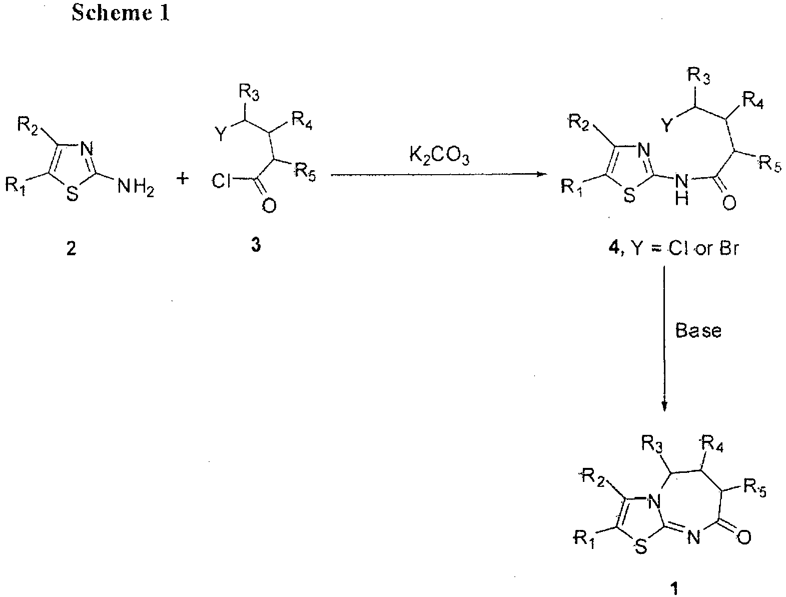 with R₁, R₂, R₃, R₄ and R₅ are each independently selected from the group consisting of hydrogen, halogen, C₁-C₂₀-alykl, C₁-C₂₀-haloalkyl, C₁-C₂₀-alkoxyl, C₁- C₂₀-haloalkoxy, aryl, heteroaryl, mercapto, alkylthio, amino, alkylamino, or wherein R₂, R₃ and R₄ are a member of a ring system.

Preferably, the reaction of compound 2 and compound 3 is in the presence of potassium carbonate in a solvent, preferably toluene, ethyl benzene, o-xylene, m-xylerie, p-xylene, octane, nonane and/or isopropyl benzene.

Even preferred, compound 4 is cyclized in the presence of at least one secondary amine, preferably diethyl amine, pyrrolidine, morpholine, piperidine and/or N-methylpiperazine, in a solvent, preferably toluene, ethylbenzene, o-xylene, m-xylene, p-xylene and/or isopropylbenzene.

The object is also achieved by a pharmaceutical composition comprising at least one of the inventive compounds and a pharmaceutically acceptable carrier or excipient.

Finally, the object is achieved by the inventive compound or the inventive pharmaceutical compositions for use anticonvulsant agent for treatment of convulsion especially for treatment of epilepsy.

Surprisingly, it was found that the compounds according the present invention possess anticonvulsant activity, preferably superior over the activity known for the state of art. In particular, it was found that compounds according to the present invention exhibit an activity 2-4 fold higher than that of valproate sodium which is used as a standard anticonvulsant agent in treatment of epilepsy.

The term "alkyl" with regard to the definition of R₁-R₅ in the compound according to formula 1 is to be understood to comprise linear and branched alkyls. The term "halo" shall comprise derivatives which are mono-, di-, tri- or poly-halosubstituted.

If possible, all substituents R₁-R₅ may be optionally further substituted, for example by halogen, amino, substituted amino, C₁-C₂₀-alkyl, C₁-C₂₀-haloalkyl, C₁-C₂₀-alkoxy of C₁-C₂₀-haloalkoxy, alkylamino, arylthio, heteroarylthio, arylamino or heteroarylamino, carboxylate, ethyl carboxylate.

Thiazolo[3,2-*a*][1,3]diazepine analogues could be obtained adopting published methods (Molina, P. et al, J. Org. Chem. 1993, 58, 5264-5270; Imming, P. et al, Arch. Pharm. (Weinheim) 1995, 238, 207-215; Lehmann, J. El-Subbagh, H. I. EI-Kashef, H. A. 2004, DE 103 20 732 A1; EI-Subbagh, H.I. et al. Bioorg. & Med. Chem let. 2008,18, 72-77). The compounds of the invention and their analogues (1) are synthesized according to an inventive method, Scheme 1. The proper 2-amino-4, 5-substituted-thiazoles (2) were acylated with the suitable acid chloride derivatives (3), where Y is chlorine or bromine, preferably bromine, and anhydrous potassium carbonate in a suitable solvent, such as, for example, toluene, ethyl benzene, o-, m-, and p-xylene, octane, nonane and isopropylbenzene, preferably toluene and ethylbenzene, at temperature ranging from room temperature to 150°C, preferably 100-120°C. The products 4 can be purified by silica gel and neutral alumina chromatography. Compounds of the formula 4 were cyclized preferably using secondary amines, such as for example, diethylamine, pyrrolidine, morpholine, piperidine, N-methylpiperazine, preferably pyrrolidine and piperidine, in a suitable solvent, such as for example toluene, ethylbenzene, o-, m-, and p-xylene, isopro-pylbenzene, preferably toluene, o-xylene at temperature ranging 100-180°C, preferably 120-130°C. The products 1 can be purified by silica gel and neutral alumina chromatography. Representative examples of such synthesis are shown in Examples 1-4 below.

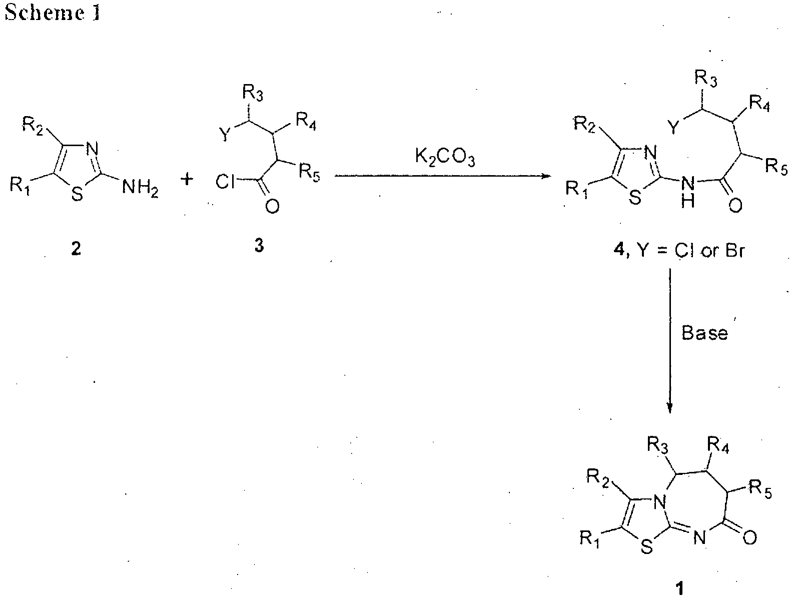

Adult male Swiss albino mice (22-28 g), approximately 10-week old, were used to conduct the anticonvulsant evaluation. They were housed in cages and kept at a temperature of 20 ± 2°C and a relative humidity of 55 ± 5% with a light-dark cycle of 12 h and fed with standard diet and water *ad libitum*. Compounds of the formula 1 have been dissolved in dimethylsulfoxide and administered at dose levels of 25, 50, 100 and 200 mgl/kg intraperitoneal (i.p.). Sodium valproate was freshly dissolved in 0.9 % NaCl and used at dose level of 200 mg/kg (1.38 mmol/kg) i.p., 30 min after the test compounds as a positive control. A control group of mice was included and received intraperitoneal (i.p.) injections of the test compounds' vehicle. Pentylenetetrazole (PTZ) was freshly dissolved in 0.9 % NaCl and used at dose level of 100 mg/kg, i.p. This dose was found to be the minimum dose that induced 100% clonic convulsion. Picrotoxin (pic) was freshly dissolved in 1 ml 0.1N warmed HCl and the final volume was made up with 0.9 % NaCl. It has been used at dose level of 10 mg/kg i.p. and was given 30 min after the test compounds.

The anticonvulsant activity of compounds of the formula 1 in mice was measured using Pentylenetetrazole (PTZ) seizure threshold test (White, H.S. et al. In Antiepileptic Drugs, 4th ed.; L,evy, R.H. et al., Eds, Raven: New York, 1995, pp 99-121). The active compounds of the formula 1 were further tested against maximal electroshock seizure (MES), and picrotoxin induced clonic convulsions, to explore the involvement of GABA-ergic receptors in their anticonvulsant activity. The effect of compounds of the formula 1 on motor performance was studied by chimney test (Dauge, V. et al. Neuropsychopharmacology 2001, 25, 690-98). Median effective (ED₅₀), and median sedative (TD₅₀) were calculated. Representative-examples are shown in Table 1, Examples 5-9.

The acute toxicity determination (LD₅₀) for compounds of formula 1 was performed. Compounds were given intraperitoneally (i.p.) in doses ranging from 0.1-5 mmol/kg. The animals. were observed for up to 6 hours continuously and were then kept under observation for 72 hours. All behavioral changes and death during the observation periods were recorded. The percentage of death at each dose level was then calculated, and the LD₅₀ values were obtained (Ghosh, M., Fundamentals of Experimental Pharmacology, Scientific Book Agency, Calcutta. 1984, pp 153-158, 187-189). The protective index (PI) and therapeutic index (TI) of each compound were calculated. Representative examples are shown in Table 1, Examples 10-11.

Biological evaluation of the new compounds of the formula 1 of the invention revealed that the compounds possess a remarkable anticonvulsant activity. The obtained results clearly point to the discovery of a new group of anticonvulsant agents that induce their actions via interaction with GABA_{A} receptors. The safety of the new compounds of the formula 1 of the invention is comparable to that of valproate sodium. Therefore, compounds of the formula 1 of the invention have the potential use as anticonvulsants, with potency proved to be 2-4 fold more active than valproate sodium.

Compounds of the formula 1 of the invention, and their acid addition salts display anticonvulsant activity. The present invention includes pharmaceutical formulations which, in addition to non-toxic, inert pharmaceutically suitable excipients, contain one or more active compounds according to the invention, or which consist of one or more active compounds according to the invention, as well as processes for the preparation of these formulations.

The present invention also includes pharmaceutical formulations in dosage units. This means that the formulations are in the form of individual parts, for example tablets, dragees, capsules, pills, and ampoules, of which the content of active compound corresponds to a fraction or a multiple of an individual dose. The dosage units can contain, for example, 1, 2, 3 or 4 individual doses or 1/2, 1/3 of 1/4 of an individual dose. An individual dose preferably contains the amount of active compound which is given in one administration and which usually corresponds to a whole, a half, a third or a quarter of a daily dose.

By non-toxic, inert pharmaceutically suitable excipients there are to be understood solid, semi-solid or liquid diluents, fillers and formulations auxiliaries of every kind.

Tablets, dragees, capsules, pills, granules, solutions and sprays may be mentioned as preferred, pharmaceutical formulations

Tablets, dragees, capsules and pills can contain the active compound or compounds alongside the customary excipients, such as (a) fillers and extenders, for example starches, lactose, sucrosse, glucose, mannitol and silica, (b) binders, for example carboxymethylcellulose, alginates, gelatin and polyvinylpyrrolidone, (C) humectants for example agar-agar, calcium carbonate and sodium bicarbonate, (e) solution retarders, for example paraffin, and (f) resorption accelerators, for example quaternary ammonium compounds, (g) wetting agents, for example cetyl alcohol and glycerol monostearate, (h) adsorbents for example kaolin and bentonite, and (i) lubricants, for example talc, calcium stearate and magnesium stearate and solid polyethylene glycols, or mixtures of the compounds listed under (a) to (i).

The tablets, dragees, capsules and pills can be provided with the customary coatings and shells, optionally containing pacifying agents, and can also be of such composition that they release the active compound or compounds only, or preferably, in a certain part of the intestinal tract, optionally in a delayed manner, examples of embedding compositions which can be used being polymeric substances and waxes.

The active compound or compounds, optionally together with one or more of the above mentioned excipients could also be in a micro-encapsulated form.

Solutions and emulsions for parenteral administration can contain, in addition to the active compound or compounds, the customary excipients, such as solvents, solubilizing agents and emulsifiers, for example, water, ethyl alcohol, isopropyl alcohol, ethyl carbonate, ethyl acetate, benzyl alcohol, benzyl benzoate, propylene glycol, 1,3-butylene glycol, dimethylformamide, oils, especially cotton seed oil, groundnut oil, maize germ oil, olive oil, caster oil and sesame oil, glycerol, glycerol-formal, tetrahydrofurfuryl alcohol, polyethylene glycol and fatty acid esters of sorbitol, or mixtures of these substances, in a sterile form which is isotonic with blood.

The therapeutically active compounds should preferably be present in the above-mentioned pharmaceutical formulations in a concentration of about 0.1 to 99.5, preferably of about 0.5 to 95% by weight of the total mixture.

The above-mentioned pharmaceutical formulations can also contain other pharmaceutical formulations and/or other pharmaceutical active compounds in addition to the active. compounds according to the invention.

The above-mentioned pharmaceutical formulations are prepared in the customary manner according to known methods, for example by mixing the active compound or compounds with the excipient or excipients.

The present invention also includes the use of the active compounds according to the invention, and of pharmaceutical formulations which contain one or more active compounds according to the invention in human and veterinary medicine.

The actual dosage unit will be determined by such generally recognized factors as body weight of the patient and/or severity and type of pathological condition the patient might be suffering. With these considerations in mind, the dosage unit for a particular patient can be readily determined by the medical practitioner in accordance with the techniques known in the medical arts.

The precise instructions for pharmaceutical administration of the compounds and agents according to the invention necessarily depend on the requirements of the individual case, the nature of treatment, and of course the opinion of the treating physician.

### Example 1

### Ethyl 2-(4-chlorobutanamido)-4-methylthiaxole-5-carboxylate.

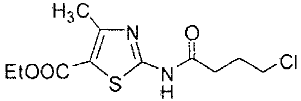

A mixture of ethyl 2-amino-4-methylthiazole-5-carboxylate(7.44 g, 0.04 mol), 4-chlorobutyryl chloride (11.3 g, 0.08 mol), and potassium carbonate (5.5 g, 0.04 mol) in toluene (100 ml) was stirred at room temperature for 24 h. The toluene was then evaporated under reduced pressure. The residue was then quenched with water, stirred, and filtered. The solid obtained was washed, dried and recrystallized. from chloroform to give the required product (8.7 g, 75% yield), mp 140-2°C, m/e 290.6, 100% (consistent with molecular formula C₁₁H₁₅ClN₂O₃S, calcd. 290.77). ¹H NMR (CDCl₃): δ 1.29 (t, 3H, *J* = 7.0 Hz, C*H*₃-CH₂-), 2.14 (t, 2H, *J*= 6.0 Hz, -CH₂-C*H*₂-CH₂-), 2.59-2.61 (m, 5H, C*H*₃- & -C*H*₂-CH₂-CH2-), 3.57 (t, 2H, *J* = 6.0 Hz, -CH₂-CH₂-CH₂-), 4.24-4.28 (q, 2H *J* **=** 7.0 Hz, CH₃-C*H*₂-), 9.92 (brs, 1H, NH). ¹³C NMR: δ 14.3, 1.7.3, 27.2, 32.8, 43.6, 61.1, 115.1, 155.6, 160.3., 162.6, 170.2.

### Example 2

### Ethyl 3-methyl-8-oxo-5,6,7,8-tetrahydro-thiarolo[3,2-a][1,3]diazepinie -2-carboxylate (SGH-35).

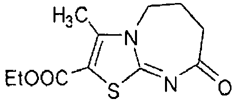

A mixture of ethyl 2-(4-chlorobutanamido)-4-methylthiazole-5-carboxylate(1.16 g, 0.004 mol) and diethylamine (0.8 ml, 0.008 mol) in toluene (50 ml) was heated under reflux for 3 h. The reaction mixture was cooled, poured into water and stirred. Toluene was separated dried and evaporated to give a crude product which was purified by repeated silica gel column chromatography eluting with CHCl₃/Hexane (90:10 v/v), The solid obtained was washed, dried and recrystallized from EtOH/H₂O to give the required product (0.41 g, 40% yield); mp 95-7°C; m/e 254.6, 100% (consistent with molecular formula C₁₁H₁₄N₂O₃S, calcd. 254.31). IR (KBr, cm⁻¹): 3026 (CH₃), 2980 (CH₂), 1707, 1695 (2 CO). ¹H NMR (CDCl₃): δ 1.26 (t, 3H, *J* = 7.0 Hz, C*H*₃-CH₂-) 2.16-2.22 (m, 2H, *J* = 7,5 Hz, -CH₂-C*H*₂-CH₂-), 2.58 (s, 3H, C*H*₃-), 2.63 (t, 2H, *J* = 8.0 Hz, -C*H*₂-CH₂-CH₂), 4.08 (t, 2H, *J* = 7.5 Hz, -CH₂-CH₂-C*H*₂-), 4.21-4.26 (q, 2H, *J* = 7.0 Hz, CH₃-C*H*₂-). ¹³C NMR: δ 14.4. 17.3, 18.1, 31.6, 47.6, 60.8, 116.00, 156.5, 158.4, 162.9, 174.0.

### Example 3

### N-(5-Bromo-4-methylthiazol-2-yl)-4-chlorobutanamide

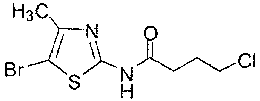

A mixture of 5-bromo-4-methylthiazol-2-amine (7.72 g, 0.04 mol), 4-chlorobutyryl chloride (11.3 g, 0.08 mol), and potassium carbonate (5.5 g, 0.04 mol) in toluene (100 ml) was stirred at room temperature for 24 h. The toluene was then evaporated under reduced pressure. The residue was then quenched with water, stirred, and filtered. The solid obtained was washed, dried and recrystallized from chloroform to give the required product (7.5 g, 63% yield), mp 112-4°C, m/e 297.6, 25% (consistent with molecular formula C₈H₁₀BrClN₂OS, calcd. 297.6). ¹H NMR (DMSO-d₆): δ 2.02-2.08 (m, 2H, *J* = 7.0 Hz, -CH₂-C*H*₂-CH₂-), 2.61-2.65 (m, 5H, C*H*₃- & -C*H*₂-CH₂-CH₂-), 3.69 (t, 2H, *J*= 7.0 Hz, -CH₂-CH₂-C*H*₂-), 12.42 (brs, 1H, NH). ¹³C NMR: δ 14.7, 27.3, 32.0, 44.7, 62.9, 158.3, 159.1, 170.4.

### Example 4

### 2-Bromo-3-methyl-6,7-dihydro-thiazolo[3,2-a][1,3]diazepin-8(5H)-one (SCH-40)

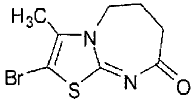

A mixture of N-(5-bromo-4-methylthiazol-2-yl)-4-chlorobutanamide(1.19 g, 0.004 mol) and diethylamine (0.8 ml, 0.008 mol) in toluene (50 ml) was heated under reflux for 3 h. The reaction mixture was cooled, poured into water and stirred. Toluene was separated dried and evaporated to give a crude product which was purified by repeated silica gel column chromatography eluting with CHCl₃/Hexane (90:10 v/v), The solid obtained was washed, dried and recrystallized EtOH/H₂O to give the required product (0.6 g, 57% yield), mp 165-7°C, m/e 261.7, 20% (consistent with molecular formula C₈H₉BrN₂OS, calcd. 261.14). IR (KBr, cm⁻¹): 3021 (CH₃), 2992 (CH₂), 1707 (CO). ¹H NMR (DMSO-d₆): δ 2.15-2.21 (m, 2H, J - 7.0 Hz, - CH₂-CH₂-CH2-), 2.60-2.65 (m, 5H, CH₃-CH₂-CH₂-CH₂-), 4.04 (t, 2H, J = 7.0 Hz, -CH₂-CH₂-CH₂-). ¹³C NMR: δ 14.9, 17.7, 30.7, 47.6, 157.3, 159.0, 159.8, 173.8.

The NMR spectral data assignments of compounds of Example 1-4 are based on analysis of the ¹H, Attached Proton Test (APT), the Distortionless Enhancement Polarization Transfer (DEPT), correlated spectroscopy (COSY), Heteronuclear Multiple Quantum Coherence Spectroscopy (HMQC), NMR spectra for each compound

### Example 5

### Effect of SGH-35 and SGH-40 on Pentylenetetrazole (PTZ)-induced convulsion

Pentylenetetrazole (PTZ) seizure threshold test is used to evaluate the potential anticonvulsant activity of compounds of the formula 1 (White, H.S. Et al. In Antiepileptic Drugs, 4th ed.; Levy, R.H. et al., Eds, Raven: New York, 1995, pp 99-121). Each compound was tested in 4 mice groups (Swiss albino mice, 25 g body weight) for each dose level (25, 50, 100 and 200 mg/kg) and was given i.p. 30 min later, animals were received PTZ (100 mg./kg, i.p.) and observed for 30 min. A single 5 seconds episode of clonic spasms was taken as a threshold seizure. SGH-35 and SGH-40 produced 100% protection against PTZ-induced seizures, Table 1.

**Table 1: ED₅₀, TD₅₀, PI, LD₅₀ and TI for SGH-35 and SGH-40.**

| **Compd³** | **Dose** | | | **% Protection** | **ED₅₀^{d} mg/kg** | **TD₅₀^{d} mg/kg** | **PI^{e}** | **LD₅₀ mg/kg** | **TI^{f}** |
|---|---|---|---|---|---|---|---|---|---|
| | **mmol/kg** | **mg/kg** | | | | | | | |
| | | | **PTZ** | 100 | 127±9.3 | 200±12.3 | 1.57 | | |
| **SGH-35** | 0.78 | **200** | **MES^{b}** | 100 | 90±5.7 | 200±12.3 | 2.22 | 670 | 5.28 |
| | | | **Pic^{c}** | 100 | 148±11.1 | 200±12.3 | 1.35 | | |
| | | | | | | | | | |
| | | | **PTZ** | 100 | 112±3.9 | 170±6.9 | 1.51 | | |
| **SGH-40** | 0.38 | **100** | **MES^{b}** | 100 | 78±4.1 | 170±6.9 | 2.17 | 640 | 5.71 |
| | | | **Pic^{c}** | 100 | 129±6.1 | 170±6.9 | 1.31 | | |

| | | | | | | | | | |
|---|---|---|---|---|---|---|---|---|---|
| ^{a} Each dose of selected compounds was tested using 4 animals and the percentage of animals protected was recorded and the anticonvulsant activity was calculated. ^{b} MES: Maximal electroshock test, was applied 30 min after the administration of each compound. ^{c} Pic:Picrotoxin (8 mg/kg, IP) was given 30 min after the administration of each compound. ^{d} ED₅₀ and TD₅₀: Median effective and median sedative doses, respectively. ^{e} PI: Protective index = TD₅₀/ED₅₀ ^{f}TI: Therapeutic index = LD₅₀/ED₅₀. | | | | | | | | | |

### Example 6

### Effect of SGH-35 and SGH-40 against the maximal electroshock (MES)-induced convulsion

The maximal electroshock (MES) seizure is one of the electrical test used to evaluate anticonvulsant activity (White, H.S. et al. In Antiepileptic Drugs, 4th ed.; Levy, R.H. et al., Eds, Raven New York, 1995, pp 99-121). MES that induced 100% maximal seizure was found to be 50 mA alternating current of 100Hz frequency for 0.2 seconds, using ECT UNIT (model number 7801, UGO Basile, Varese, Italy). Swiss albino mice (25 g body weight) were injected with 25, 50, 100 and 200 mg/kg of SGH-35 and SGH-40 i.p. 30 min later, mice were restrained by hand and subjected to electric shock through their ears, and released immediately following electrical stimulation, to permit observation of the maximal seizure. The maximal seizure typically consists of a short period of initial tonic flexioni and a prolonged period of tonic extension (especially the hind limb). Protection was defined as complete absence of hind limb tonic extension (Table 1).

### Example 7

### Effect of SGH-35 and SGH-40 against picrotoxin (pic)-induced convulsion

Swiss albino mice (25 g body weight) were injected with 25, 50, 100 and 200 mg/kg of SGH-35 and SGH-40 (4 mice, i.p.). 30 min later, animals were injected sc with Pic (10 mg/kg) and observed for 30 min and the percentage protection against convulsive seizures for each compound was calculated (Table I).

### Example 8

### Minimal neurotoxicity (chimney test) for SGH-35 and SGH-40

The effect of SGH-35 and SGH-40 on motor performance was studied by chimney test (Dauge, V. et al. Neuropsychopharmacology 2001, 25, 690-98). At 15 and 25 min after i.p. administration of compounds SGH-35 and SGH-40, the inability of mice to climb up backwards in a glass tube of 25 cm length and 3 cm inner diameter within 30 sec was recorded and taken as a measure of neurological deficits. Normal mice climb up in 5-10 sec. The dose that impaired the motor coordination in 50% of the animals tested was labeled as the median toxin dose 50 (TD₅₀).

### Example 9

### Calculation of the anticonvulsant ED₅₀ values for SGH-35 and SGH-40

In each of the chemical and the electrical methods described above, Swiss albino mice were divided into 4 groups (N = 4 animals). Each group was injected (i.p.) with one of the four selected doses that have proven to obtain a variable percentage of protection 20 minutes before the convulsants. The effective dose 50 (ED₅₀) i.e. the dose that protected half of the animals was then calculated using the log-probit method (White, H.S. et al. In Antiepileptic Drugs, 4th ed.; Levy, R.H.et al., Eds, Raven: New York, 1995, pp 99-121).

### Example 10

### Determination of LD₅₀ and dose-response curve:

Initially the approximate LD₁₀₀ of each test compound was determined. Albino Swiss mice (25 g body weight) were then divided into 4 groups (6 mice each). Each group was then injected with one of 4 selected doses the highest being the LD₁₀₀ dose. The animals were then observed closely for 4 hours and then occasionally for 72 hours. The percentage of death in each group was then calculated. The LD₅₀ doses were then calculated using the Profit method (Ghosh; M., Fundamentals of Experimental Pharmacology, Scientific Book Agency, Calcutta. 1984, pp 153-158, 187-189).

### Example 11

### Calculation of the protective Index (PI) and Therapeutic Index for SGH-35 and SGH-40

The protective index (PI) - i.e. the value that indicates the margin of safety and tolerance of the test compound was calculated by the formula: Protective index (PI) = TD₅₀/ED₅₀. The Therapeutic Index was calculated by the formula: Therepeutic index (TI) = LD₅₀/ED₅₀ (Löscher, W. et al. Epilepsy Res. 1991, 9, 1-10).

The features disclosed in the foregoing description and in the claims may, both separately and in any combination thereof, be material for realizing the invention in diverse forms thereof.

## Claims

1. Compound according to formula 1: 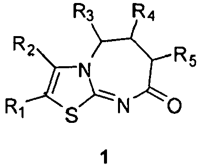 wherein R₁, R₃, R₄ and R₅ are each independently selected from the group consisting of hydrogen, halogen, C₁-C₂₀-alkyl, C₁-C₂₀-haloalkyl, C₁-C₂₀-alkoxy, C₁-C₂₀-haloalkoxy, aryl, heteroaryl, mercapto, alkylthio, amino, alkylamino, R₂ is selected from the group consisting of halogen, C₁-C₂₀-alkyl, C₁-C₂₀-haloalkyl, C₁-C₂₀-alkoxy, C₁-C₂₀-haloalkoxy, aryl, heteroaryl, mercapto, alkylthio, amino, alkylamino or wherein R₃ and R₄ are a member of a ring system.

2. Compound according to claim 1, wherein R₂ is selected from the group consisting of mercapto and C₁-C₂₀-alkyl, halogen or amino.

3. Compound according to claim 1 or 2, wherein R₃ is hydrogen or is taken together with R₄ to form an alicyclic, aryl or heteroaryl ring system.

4. Compound according to any of the preceding claims, wherein the compound is present in form of its addition salt.

5. Compound according to claim 4, wherein the compound is present in form of its hydrochloride, hydrobromide, phosphate, nitrate, acetate, malate, succinate, fumarate, tartrate, salicylate, sorbate, lactate, p-toluene sulphate, or naphthalene-1,5-disulfonate salts.

6. Method for preparing a compound according to claim I, comprising reacting a compound according to formula 2 with a compound according to formula 3 to prepare a compound according to formula 4, and reacting compound of formula 4 to result in compounds of formula 1, as given in scheme 1: 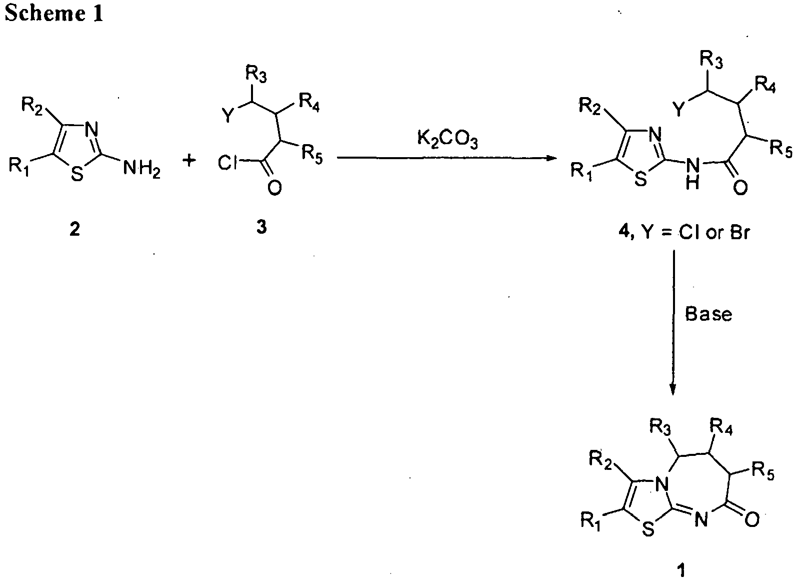 with R₁, R₂, R₃, R₄ and R₅ as defined in claim 1.

7. Method according to claim 6, wherein the reaction of compound 2 and compound 3 is in the presence of potassium carbonate in a solvent.

8. Method according to claim 7, wherein the reaction of compound 2 and compound 3 is in the presence of potassium carbonate in toluene, ethyl benzene, o-xylene- m-xylene, p-xylene, octane, nonane and/or isopropyl benzene.

9. Method according to claim 6 to 8, wherein compound 4 is cyclized in the presence of at least one secondary amine in a solvent.

10. Method according to claim 9, wherein the reaction of compound 2 and compound 3 is in the presence of diethyl amine, pyrrolidine, morpholine, piperidine and/or N-methylpiperazine in a solvent.

11. Pharmaceutical composition comprising at least one compound according to any of the claims 1-5 and a pharmaceutically acceptable carrier or excipient.

12. Compound according to any of the claims 1-5 or the pharmaceutical composition according to claim 11 for use as anticonvulsant agent in_treatment of convulsion, especially in treatment of epilepsy.

## Patentansprüche

1. Verbindung gemäß der Formel 1: 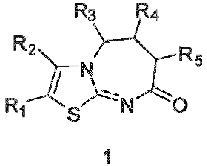 wobei R₁, R₃, R₄ und R₅ jeweils unabhängig ausgewählt sind aus der Gruppe, bestehend aus Wasserstoff, Halogen, C₁-C₂₀-Alkyl, C₁-C₁₀-Halogenalkyl, C₁-C₂₀-Alkoxy, C₁-C₂₀-Halogenalkoxy, Aryl, Heteroarly, Mercapto, Alkylthio, Amino, Alkylamino, R₂ ausgewählt ist aus der Gruppe, bestehend aus Halogen, C₁-C₂₀-Alkyl, C₁-C₂₀-Halogenalkyl, C₁-C₂₀-Alkoxy, C₁-C₂₀-Halogenalkoxy, Aryl, Heteroaryl, Mercapto, Alkylthio, Amino, Alkylamino oder wobei R₃ und R₄ ein Teil eines Ringsystems sind.

2. Verbindung nach Anspruch 1, wobei R₂ ausgewählt ist aus der Gruppe, bestehend aus Mercapto und C₁-C₂₀-Alkyl, Halogen oder Amino.

3. Verbindung nach Anspruch 1 oder 2, wobei R₃ Wasserstoff ist oder zusammen mit R₄ ein alicyklisches, Aryl- oder Heteroarylringsystem bildet.

4. Verbindung nach einem der vorangehenden Anspruche, wobei die Verbindung in Form ihres Additionssalzes vorliegt

5. Verbindung nach Anspruch 4, wobei die Verbindung in Form ihres Hydrochlorid, Hydrobromid, Phosphat, Nitrat, Acetat, Malat, Succinat, Fumarat, Tartrat, Salicylat, Sorbat, Lactat, p-Toluolsulphat oder Naphthalen-1,5-Disulfonatsalzes gemäß Anspruch 5 vorliegt.

6. Verfahren zur Herstellung einer Verbindung nach Anspruch 1, umfassend das Reagieren einer Verbindung gemäß Formel 2 mit einer Verbindung gemäß Formel 3, um eine Verbindung gemäß Formel 4 herzustellen und reagieren der Verbindung gemäß Formel 4, um eine Verbindung der Formel 1 zu erhalten, wie im Schema 1 dargestellt: 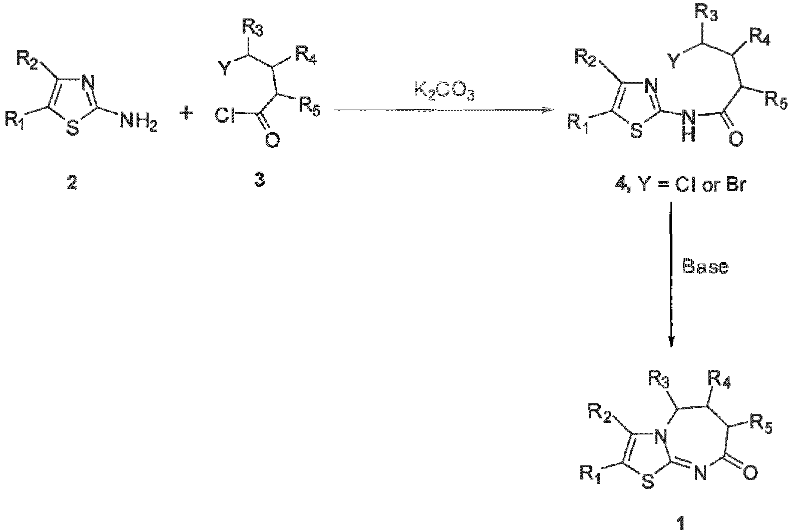 wobei R₁, R₂, R₃, R₄ und R₅ wie in Anspruch 1 definiert sind.

7. Verfahren nach Anspruch 6, wobei die Reaktion der Verbindung 2 und der Verbindung 3 in Gegenwart von Kaliumcarbonat in einem Lösungsmittel durchgeführt wird.

8. Verfahren nach Anspruch 7, wobei die Reaktion der Verbindung 2 und der Verbindung 3 in Gegenwart von Kaliumcarbonat in Toluol, Ethylbenzol, o-Xylol, m-Xylol, p-Xylol, Octan, Nonan und/oder Isopropylbenzol durchgeführt wird.

9. Verfahren nach einem der Ansprüche 6 bis 8, wobei die Verbindung 4 in Gegenwart zumindest eines sekundären Amins in einem Lösungsmittel cyclisiert wird.

10. Verfahren nach Anspruch 9, wobei die Reaktion der Verbindung 2 und der Verbindung 3 in Gegenwart von Diethylamin, Pyrrolidin, Morpholin, Piperidin und/oder N-methylpiperazin in einem Lösungsmittel durchgeführt wird.

11. Pharmazeutische Zusammensetzung, umfassend zumindest eine Verbindung nach einem der Ansprüche 1 bis 5 und einen pharmazeutisch akzeptablen Träger oder Hilfsstoff.

12. Verbindung nach einem der Ansprüche 1 bis 5 oder die pharmazeutische Zusammensetzung nach Anspruch 11 zur Verwendung als Antikonvulsant in der Behandlung von Konvulsionen, vorzugsweise in der Behandlung von Epilepsie.

## Revendications

1. Composé selon la formule 1 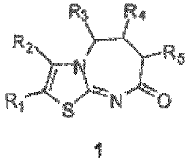 dans laquelle formule R₁, R₃, R₄ et R₅ sont chacun indépendamment sélectionnés dans le groupe constitué d'hydrogène, d'halogène, d'alkyle en C₁ à C₂₀, d'haloalkyle en C₁ à C₂₀, d'alcoxy en C₁ à C₂₀, d'haloalcoxy en C₁ à C₂₀, d'aryle, d'hétéroaryle, de mercapto, d'alkylthio, d'amino, d'alkylamino, R₂ est sélectionné dans le groupe constitué d'halogène, d'alkyle en C₁ à C₂₀, d'haloalkyle en C₁ à C₂₀, d'alcoxy en C₁ à C₂₀, d'haloalcoxy en C₁ à C₂₀, d'aryle, d'hétéroaryle, de mercapto, d'alkylthio, d'amino, d'alkylamino ou dans laquelle formule R₃ et R₄ appartiennent à un système cyclique.

2. Composé selon la revendication 1, dans lequel R₂ est sélectionné dans le groupe constitué de mercapto et d'alkyle en C₁ à C₂₀, d'halogène ou d'amino.

3. Composé selon la revendication 1 ou la revendication 2, dans lequel R₃ représente l'hydrogène ou est pris conjointement avec R₄ pour former un système cyclique alicyclique, aryle ou hétéroaryle.

4. Composé selon l'une quelconque des revendications précédentes, dans lequel le composé est présent sous la forme de son sel d'addition.

5. Composé selon la revendication 4, le composé étant présent sous la forme de ses sels d'hydrochlorure, d'hydrobromure, de phosphate, de nitrate, d'acétate, de malate, de succinate, de fumarate, de tartrate, de salicylate, de sorbate, de lactate, de sulfate de p-toluène ou de naphtalène-1,5-disulfonate.

6. Procédé de préparation d'un composé selon la revendication 1, comprenant la réaction du composé selon la formule 2 avec un composé selon la formule 3 pour préparer un composé selon la formule 4 et la réaction du composé de la formule 4 pour produire les composés de la formule 1, comme indiqué dans le schéma 1 : 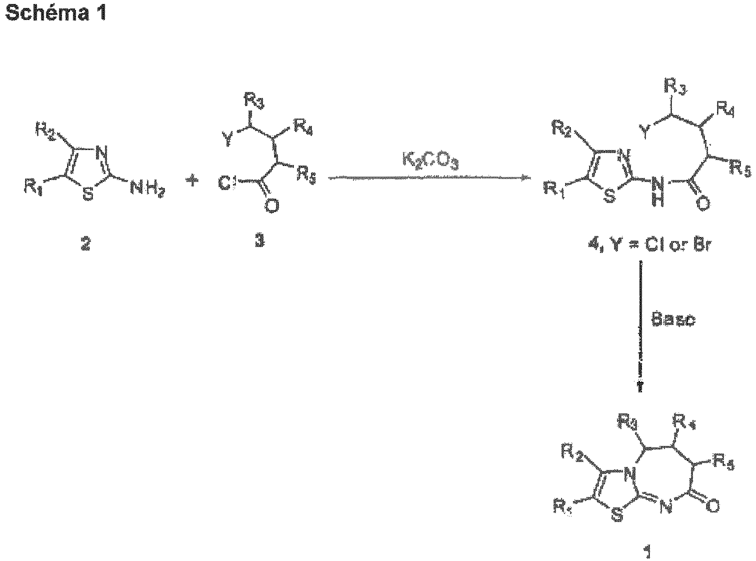 4, Y = Cl ou Br
Base
avec R₁, R₂, R₃, R₄ et R₅ comme défini dans la revendication 1.

7. Procédé selon la revendication 6, dans lequel la réaction du composé 2 et du composé 3 a lieu en présence de carbonate de potassium dans un solvant.

8. Procédé selon la revendication 7, dans lequel la réaction du composé 2 et du composé 3 a lieu en présence de carbonate de potassium dans du toluène, de l'éthylbenzène, de l'o-xylène-m-xylène, du p-xylène, de l'octane, du nonane et/ou de l'isopropylbenzène.

9. Procédé selon la revendication 6 à 8, dans lequel le composé 4 est cyclisé en présence d'au moins une amine secondaire dans un solvant.

10. Procédé selon la revendication 9, dans lequel la réaction du composé 2 et du composé 3 a lieu en présence de diéthylamine, de pyrrolidine, de morpholine, de pipéridine et/ou de N-méthylpipérazine dans un solvant.

11. Composition pharmaceutique comprenant au moins un composé selon l'une quelconque des revendications 1 à 5 et un support ou excipient pharmaceutiquement acceptable.

12. Composé selon l'une quelconque des revendications 1 à 5 ou la composition pharmaceutique selon la revendication 11 destiné(e) à une utilisation comme agent anticonvulsivant dans le traitement des convulsions, en particulier dans le traitement de l'épilepsie.
